(19) Europäisches Patentamt
European Patent Office
Office européen des brevets

(11) Veröffentlichungsnummer: **0 087 596**
**B1**

(12) # EUROPÄISCHE PATENTSCHRIFT

(45) Veröffentlichungstag der Patentschrift: **24.10.84**

(51) Int. Cl.³: **C 07 C 49/16**, C 07 C 69/06, C 07 C 45/59

(21) Anmeldenummer: **83100865.1**

(22) Anmeldetag: **31.01.83**

(54) **Verfahren zur Herstellung von 5-substituierten 1-Chlor-3,3-dimethylpentan-2-onen.**

(30) Priorität: **11.02.82 DE 3204788**

(43) Veröffentlichungstag der Anmeldung: **07.09.83 Patentblatt 83/36**

(45) Bekanntmachung des Hinweises auf die Patenterteilung: ***24.10.84 Patentblatt 84/43***

(84) Benannte Vertragsstaaten: **CH DE FR GB LI**

(56) Entgegenhaltungen:
**DE - A - 2 216 838**
**DE - B - 2 716 896**
**DE - C - 828 540**

(73) Patentinhaber: **BAYER AG, Konzernverwaltung RP Patentabteilung, D-5090 Leverkusen 1 Bayerwerk (DE)**

(72) Erfinder: **Jautelat, Manfred, Dr., Müllersbaum 28, D-5093 Burscheid (DE)**

EP 0 087 596 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft ein neues Verfahren zur Herstellung von 5-substituierten 1-Chlor-3,3-dimethylpentan-2-onen, die als Zwischenprodukte zur Herstellung von fungizid und antimykotisch wirksamen Verbindungen dienen können.

Es wurde gefunden, dass man bisher schwer zugängliche 5-substituierte 1-Chlor-3,3-dimethylpentan-2-one der allgemeinen Formel:

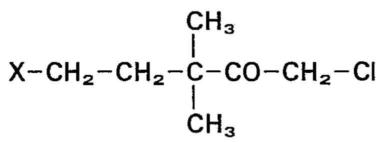

(I)

in der

X für Halogen oder einen Acyloxyrest mit 1 bis 8 Kohlenstoffatomen steht, in guten Ausbeuten dadurch herstellen kann, dass man 2-Chlormethylen-3,3-dimethyltetrahydrofuran der Formel:

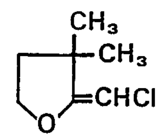

(II)

mit aciden Verbindungen der Formel:

$$H-X \qquad (III)$$

in welcher

X die oben genannte Bedeutung hat,

bei Temperaturen von 0 bis 200° C umsetzt.

Es ist als ausgesprochen überraschend zu bezeichnen, dass die Umsetzung mit Halogenwasserstoffen oder Carbonsäuren nicht zu einer 1,2-Addition an die Doppelbindung führt, sondern unter Ringöffnung 5-substituierten 1-Chlor-3,3-dimethylpentan-2-one der Formel I ergibt. Das Verfahren ist daher von technischem Interesse.

Das erfindungsgemässe Verfahren weist eine Reihe von Vorteilen auf. So verläuft die Umsetzung in hoher Selektivität und Ausbeute. Die Reaktionsbedingungen sind mild und schonend.

Verwendet man 2-Chlormethylen-3,3-dimethyltetrahydrofuran und Chlorwasserstoff als Ausgangsstoffe, so kann die Reaktion durch das folgende Formelschema wiedergegeben werden:

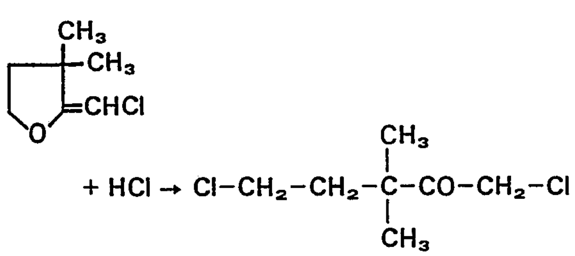


2-Chlormethylen-3,3-dimethyltetrahydrofuran (II) ist noch nicht bekannt. Seine Herstellung erfolgt aus dem bekannten 1,1,5-Trichlor-3,3-dimethyl-1-penten (DE-OS Nr. 3029970) durch sukzessive Umsetzung mit Carboxylaten und Basen in einem Eintopfverfahren gemäss folgendem Reaktionsschema

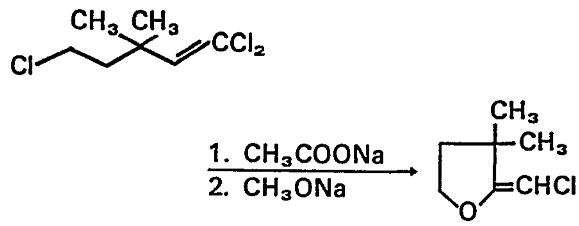


Die verwendeten Ausgangsstoffe der Formel III sind allgemein bekannt. In der Formel III steht X für Halogen, vorzugsweise Chlor, Brom, Jod, oder Acyloxy mit 1 bis 8 C-Atomen, vorzugsweise Formyloxy, Acetoxy, Chloracetoxy, Benzoyloxy, 2,4-Dichlorbenzoyloxy.

Die Reaktionkann sowohl in Abwesenheit als auch in Gegenwart eines Verdünnungsmittels durchgeführt werden. Als Verdünnungsmittel kommen alle inerten organischen Lösungsmittel in Frage. Beispielsweise Kohlenwasserstoffe wie Toluol oder Chlorbenzol, halogenierte Kohlenwasserstoffe wie Methylenchlorid oder Chloroform, oder Ether wie 1,2-Dimethoxyethan oder Dioxan.

Die Reaktionstemperatur kann in einem grösseren Bereich variiert werden. Im allgemeinen arbeitet man zwischen 0 und 200, vorzugsweise zwischen 20 und 150° C. Die Umsetzung kann sowohl bei Normaldruck im offenen System als auch unter Druck im Autoklaven ausgeführt werden.

Als Katalysatoren, vorzugsweise bei der Umsetzung mit Carbonsäuren, kommen stark saure Stoffe wie Mineralsäuren, z.B. Schwefelsäure oder Salzsäure, Sulfonsäure wie Methansulfonsäure oder p-Toluolsulfonsäure, starke Carbonsäuren wie Trifluoressigsäure, oder Lewis-Säure wie Aluminiumchlorid in Frage. Die Katalysatoren werden nur in Mengen von 0,1 bis 5% verwendet.

Bei der Durchführung des erfindungsgemässen Verfahrens setzt man 1 bis 3 Eq der aciden Verbindung der Formel III, vorzugsweise 1 bis 2 Eq, zu einem Äquivalent des Ausgangsstoffs II ein. Die acide Verbindung kann aber auch als Verdünnungsmittel genutzt werden.

Das nach dem erfindungsgemässen Verfahren zugängliche 1,5-Dichlor-3,3-dimethylpentan-2-on ist ein geeignete Zwischenprodukt zur Herstellung von fungizid und antimykotisch wirksamen Azolderivaten. Setzt man es zum Beispiel mit Phenolen der allgemeinen Formel:

$$H-O-R$$

in welcher R für gegebenenfalls substituiertes Phenyl steht, um, so erhält man Chloretherketone der allgemeinen Formel:

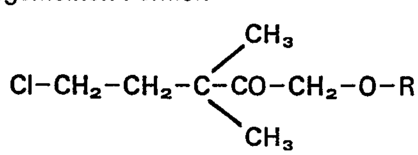

Deren Umsetzung nach Halogenierung führt mit Imidazol oder 1,2,4-Triazol in Gegenwart eines Verdünnungsmittels und eines Säurebindemittels zu Azolylphenoxytetrahydrofuran-2-ylidenmethanen der Formel:

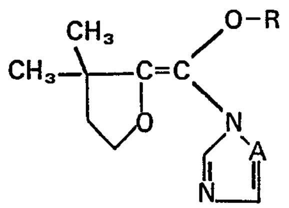

in welcher

A für ein Stickstoffatom oder die CH-Gruppe steht, und

R für gegebenenfalls substituiertes Phenyl steht.

Von diesen zeichnen sich z.B. das (4-Chlorphenoxy)-(imidazol-1-yl)-3,3-dimethyltetrahydrofuran-2-ylidenmethan, F. 85 bis 88° C, und das (4-Fluorphenoxy)-(imidazol-1-yl)-3,3-dimethyltetrahydrofuran-2-ylidenmethan, Fp. 214° C, durch sehr gute antimykotische Wirkungen aus.

*Beispiel 1:*

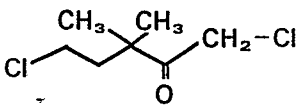

In 476 g (3,25 mol) 2-Chlormethylen-3,3-dimethyltetrahydrofuran wird unter Eiskühlung ein starker Chlorwasserstoffgasstrom aus einer Bombe eingeleitet. Das Gas wird vollständig absorbiert und die Innentemperatur steigt bis auf 30° C. Nach vollständiger Sättigung mit Chlorwasserstoff wird das Reaktionsgemisch 2 h bei Raumtemperatur nachgerührt. Überschüssiger Chlorwasserstoff wird zunächst in die Wasserstrahlpumpe gezogen, dann wird bei gutem Vakuum destilliert. Man erhält bei $Kp._{0,3}$ 85 bis 90° C 531 g (2,93 mol, 90%) 1,5-Dichlor-3,3-dimethylpentan-2-on.

NMR ($CDCl_3$): δ 1,25 (s, 6H), 2,1 (t, 2H), 3,5 (t, 2H), 4,4 (s, 2H).

*Beispiel 2:*

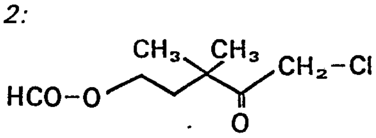

29,2 g (0,2 mol) 2-Chlormethylen-3,3-dimethyltetrahydrofuran werden in 100 ml Ameisensäure 5 h auf 60° C erhitzt. Die fraktionierte Destillation liefert bei $Kp._{0,2}$ 110° C 34 g (0,174 mol, 87%) 1-Chlor-3,3-dimethyl-5-formyloxypentan-2-on.

NMR ($CDCl_3$): δ 1,25 (s, 6H), 2,0 (t, J=7 Hz, 2H), 4,2 (t, J=7 Hz, 2H), 4,45 (s, 2H), 8,0 (s, 1H).

*Beispiel 3:*

Herstellung des Ausgangsstoffes 2-Chlormethyl-3,3-dimethyltetrahydrofuran der Formel:

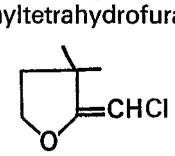

806 g (4 mol) 1,1,5-Trichlor-3,3-dimethyl-1-penten werden mit 360 g (4,4 mol) wasserfreiem Natriumacetat in 1 l Dimethylformamid 6 h unter Rückfluss erhitzt. Nach Abkühlen auf ca. 100° C tropft man 1,6 l (8 mol) 30% Natriummethylatlösung in Methanol ein und erhitzt weitere 4 h unter Rückfluss. Die kalte Lösung wird in Wasser gegossen und mit Methylenchlorid mehrfach extrahiert. Nach Trocknen der Lösung und Abtreiben des Lösungsmittels bleiben 654 g Produkt zurück, das über eine Kolonne fraktioniert wird. Bei $Kp._{20}$ 84 bis 87° C gewinnt man 522 g (3,56 mol, 89%) 2-Chlormethylen-3,3-dimethyletetrahydrofuran.

NMR ($CDCl_3$): δ 1,2 (s, 6H), 1,9 (t, J=6,5 Hz), 4,15 (t, J=6,5 Hz, 2H), 4,85 (s, 1H).

## Patentansprüche

1. Verfahren zur Herstellung von 5-substituierten 1-Chlor-3,3-dimethylpentan-2-onen der Formel:

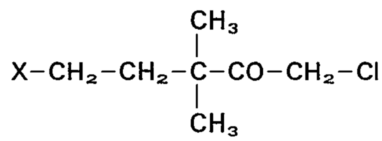 (I)

in welcher

X für ein Halogen oder einen Acyloxyrest mit 1 bis 8 Kohlenstoffatomen steht,

dadurch gekennzeichnet, dass man 2-Chlormethylen-3,3-dimethyltetrahydrofuran der Formel:

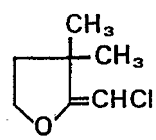 (II)

mit aciden Verbindungen der Formel:

$$H-X \quad (III)$$

in welcher

X die oben genannte Bedeutung hat,

bei Temperaturen von 0 bis 200° C umsetzt.

2. Verfahren nach Anspruch 1, dadurch gekennzeichnet, dass man die Umsetzung in Gegenwart eines Verdünnungsmittels durchführt.

3. Verfahren nach den Ansprüchen 1 und 2, dadurch gekennzeichnet, dass man als Verdünnungsmittel ein inertes organisches Lösungsmittel verwendet.

4. Verfahren nach Anspruch 1, dadurch gekennzeichnet, dass man die Umsetzung in Gegenwart eines Katalysators durchführt.

5. Verfahren nach den Ansprüchen 1 und 4, dadurch gekennzeichnet, dass man als Katalysatoren Mineralsäuren, Sulfonsäuren, starke Carbonsäuren oder Lewis-Säuren verwendet.

6. Verfahren nach den Ansprüchen 1, 4 und 5, dadurch gekennzeichnet, dass man die Katalysatoren in Mengen von 0,1 bis 5% verwendet.

7. Verfahren nach Anspruch 1, dadurch gekennzeichnet, dass man die Umsetzung bei 20 bis 150° C durchführt.

8. Verfahren nach Anspruch 1, dadurch gekennzeichnet, dass man als acide Verbindung Chlorwasserstoff verwendet.

9. Verfahren nach Anspruch 1, dadurch gekennzeichnet, dass man als acide Verbindung Ameisensäure verwendet.

10. 1,5-Dichlor-3,3-dimethylpentan-2-on.

**Claims**

1. Process for preparing 5-substituted 1-chloro-3,3-dimethylpentan-2-ones of the formula:

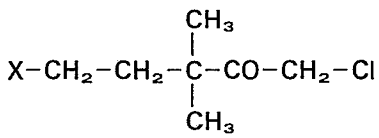 (I)

in which X represents a halogen or an acyloxy radical having 1-8 carbon atoms, characterised in that 2-chloromethylene-3,3-dimethyltetrahydrofuran of the formula:

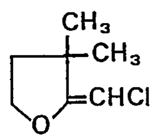 (II)

is reacted with acidic compounds of the formula:

H−X (III)

in which X has the above-mentioned meaning, at temperature of 0 to 200° C.

2. Process according to Claim 1, charaterised in that the reaction is carried out in the presence of a diluent.

3. Process according to Claims 1 and 2, characterised in that an inert organic solvent is used as the diluent.

4. Process according to Claim 1, characterised in that the reaction is carried out in the presence of a catalyst.

5. Process according to Claims 1 and 4, characterised in that mineral acids, sulphonic acids, strong carboxylic acids or Lewis acids are used as catalysts.

6. Process according to Claims 1, 4 and 5, characterised in that the catalysts are used in amounts of 0,1 to 5%.

7. Process according to Claim 1, characterised in that the reaction is carried out at 20 to 150° C.

8. Process according to Claim 1, characterised in that hydrogen chloride is used as the acidic compound.

9. Process according to Claim 1, characterised in that formic acid is used as the acidic compound.

10. 1,5-Dichloro-3,3-dimethylpentan-2-one.

**Revendications**

1. Procédé pour la fabrication de 1-chloro-3,3-diméthylpentane-2-ones 5-substituées de formule:

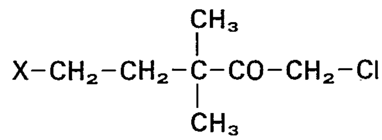 (I)

dans laquelle X représente un halogène ou un reste alcoxy ayant 1 à 8 atomes de carbone, caractérisé en ce que l'on fait réagir le 2-chlorméthylène-3,3-dimethyltétrahydrofuranne de formule:

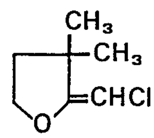 (II)

avec des composés acides de formule:

H−X (III)

dans laquelle X a la signification mentionnée ci-dessus, à des températures de 0 à 200° C.

2. Procédé selon la revendication 1, caractérisé en ce que l'on effectue la réaction en présence d'un diluant.

3. Procédé selon les revendications 1 et 2, caractérisé en ce que l'on utilise comme diluant un solvant organique inerte.

4. Procédé selon la revendication 1, caractérisé en ce que l'on effectue la réaction en présence d'un catalyseur.

5. Procédé selon les revendications 1 à 4, caractérisé en ce que l'on utilise comme catalyseurs des acides minéraux, des acides sulfoniques, des acides carboxyliques forts ou des acides de Lewis.

6. Procédé selon les revendications 1, 4 et 5, caractérisé en ce que l'on utilise les catalyseurs en quantités de 0,1 à 5%.

7. Procédé selon la revendication 1, caractérisé en ce que l'on effectue la réaction à 20-150° C.

8. Procédé selon la revendication 1, caractérisé en ce que l'on utilise comme composé acide le chlorure d'hydrogène.

9. Procédé selon la revendication 1, caractérisé en ce que l'on utilise comme composé acide, l'acide formique.

10. La 1,5-dichloro-3,3-diméthylpentane-2-one.